# EUROPEAN PATENT SPECIFICATION

(11) **EP 4 243 923 B1**
(45) Date of publication and mention of the grant of the patent: **06.08.2025**
(21) Application number: 21806292.5
(22) Date of filing: 16.11.2021
(51) Int. Cl.: A61N 1/05

(54) **ACTIVE FIXATION LEAD**
LEITUNG ZUR AKTIVEN FIXIERUNG
CONDUCTEUR DE FIXATION ACTIF

(30) Priority: 16.11.2020 FR 2011721; 16.06.2021 EP 21305824
(43) Date of publication of application: 20.09.2023
(73) Proprietor: Sorin CRM SAS, 92140 Clamart (FR)
(72) Inventor: OLLIVIER, Jean-François, 91190 Gif-sur-Yvette (FR)
(74) Representative: Ungerer, Olaf
(86) International application number: PCT/EP2021/081892
(87) International publication number: WO 2022/101513

(56) References cited:
- US-A- 4 628 943
- US-A- 5 354 327
- US-A1- 2010 179 629
- US-A1- 2010 331 943
- US-A1- 2013 245 732
- US-A1- 2017 239 464
- US-B1- 6 489 562
- US-B1- 6 687 550

## Description

### Field of Invention

The present invention relates to an implantable lead configured to be connected to a pacemaker or a defibrillator and comprising an active fixation for securing an electrode to cardiac tissue.

### Background of the invention

It is known that active fixation leads comprise an extendable and retractable mechanism for deploying a helix electrode at a distal end of the lead. In the known extendable and retractable mechanism, the helix electrode is electrically connected to and supported by an electrically conductive driver shaft movably disposed within a distal housing portion of the lead. The helix electrode is thus electrically connected to a distal coil conductor of the lead by means of the conductive driver. The helix electrode could be in intermittent contact with an internal wall of the distal housing portion of the lead in which is it movably housed.

The distal housing portion of the lead is usually a cylindrical molded plastic part. However, the distal housing portion of the lead may be provided with metallic part(s).

The presence of metal part in the distal housing portion of the lead involves that if the helix electrode and the metallic part of the housing are not continuously electrically connected, electrical chatter noises may be generated. Indeed, chatter noise may result from the intermittent contacts of these metallic parts of the lead, especially as the helix electrode or the distal metallic part of the housing is in contact with blood, being an ionic solution. Chatter noise is a parasite electrical signal that can cause the delivery of inappropriate therapy or shock.

For addressing the electrical chatter noise problem occurring between a metallic housing collar of the lead and the helix electrode, the document US 6,687,550 B1discloses the use of a compression metallic coil spring or a "ball seal" type contact spring. According to the document US 6,687,550 B1, such spring is positioned around the conductive driver, thereby generating an electrical contact between the spring and the driver, the driver being electrically connected to the helix electrode and the distal coil conductor of the lead. In particular, in the document US 6,687,550 B1, a garter spring (i.e. a circular spring) is housed in the annular recess of an annular track member disposed between the driver and a tubular conductive coupling. The annular track member according to the document US 6,687,550 B1 allows radially compressing the garter spring over the entire periphery of the driver.

However, the radial compression of the garter spring over the entire periphery of the driver increases the friction between the spring and the driver, which may generate parasitic mechanical effects. Indeed, the mechanical friction between the spring and the driver can affect the smoothness of the driver's movement, and makes the driver moves in a jerky manner. Such parasitic friction will thus jeopardize the quality of the helix screw deployment.

Moreover, in the known pacing and/or defibrillation lead, the distal housing portion of the lead comprising the retractable fixation mechanism and an anode provided at the distal end of the lead constitutes the most rigid portion of the distal part of the lead. In particular, there is the type of lead wherein said "rigid" portion is a one-piece portion completely rigid and about 14 to 16 mm long. There is also a type of lead wherein said "rigid" portion actually comprises two distinct rigid sections separated by a more flexible portion having a length between 1 and 3 mm.

The presence of a flexible portion, as mentioned above with respect to the second type of lead, is generally preferred by clinicians because it allows facilitating the implantation of the lead, in particular in the neighboring of the heart chambers and for passing the tricuspid valve. On the other hand, because the short flexible portion (having a length of 1 to 3 mm) is arranged between two more rigid portions of the lead, it may impact the long-term reliability of the lead. Indeed, the mechanical stress generated by the cyclic cardiac contractions is mostly absorbed by the short flexible section of 1 and 3 mm length.

Further known implantable pacing and/or defibrillation lead are shown in US 2010/179629 A1, US 4 628 943, US 6 489 562 B1, US 2017/239464 A1 and US 2010/331943 A1.

Hence, an object of the present invention is to provide a pacing and/or defibrillation lead comprising a retractable fixation mechanism wherein the rigid distal portion of the lead has a reduced length compared to that of the leads from the state of the art, in order to facilitate its placement.

There is also a need for improving the sealing properties of the known pacing and/or defibrillation lead wherein the distal housing portion of the lead comprises the retractable fixation mechanism. Indeed, fluid, like blood, can enter the lumen of the distal housing portion of the lead via the distal opening through which the fixation mechanism can be extended and retracted. As blood is an ionic solution, surface and/or volume corrosion can occur inside the distal end of the lead.

Hence, another object of the present disclosure is to improve the sealing properties of the distal end portion of an active fixation lead and, in particular, provide as sealing solution that allows an easy assembly of the lead.

### Description of the Invention

The present invention is defined by the appended claims.

### SUMMARY OF THE PRESENT DISCLOSURE

The present disclosure relates to of an implantable pacing and/or defibrillation lead. Said lead extending from a proximal end to a distal end, wherein the proximal end of the lead is configured to be connected to an implantable medical device, the lead comprising: - an active fixation electrode at the distal end of the lead, which is mechanically and electrically coupled to a conductive driver shaft moveably arranged in a distal housing portion of the lead, wherein the active fixation electrode is movable from a retracted position in which the active fixation electrode is completely housed inside the distal housing, to an extended position, in which the active fixation electrode extends in a distal direction at least partially beyond the distal housing portion, and - an electrically conductive compression spring. The lead further comprises a guiding member arranged inside the distal housing portion and comprising a tubular body electrically connected with the distal housing portion, wherein the conductive driver shaft is slideably arranged through a lumen extending through the tubular body, wherein the tubular body further comprises a radial opening in communication with the lumen, and wherein the electrically conductive compression spring is at least partially radially arranged around the tubular body and its radial opening, such that the electrically conductive compression spring is forced by its restoring force against the conductive driver shaft.

Hence, the occurrence of chatter noise can be avoided by means of a continuous and stable electrical contact provided between the active fixation electrode and the distal housing portion that brings the active fixation electrode and the distal housing portion onto the same electrical potential. The continuous electrical contact is realized by the electrically conductive compression spring forced by its restoring force against the conductive driver shaft.

The spring provides multi-point contacts with the conductive driver shaft at the radial opening by means of the spring's individual coils. As the electrically conductive compression spring can only contact the conductive driver shaft at the radial opening of the tubular body, friction between the electrically conductive compression spring and the conductive driver shaft may only occur at the radial opening, and not over the entire periphery of the conductive driver shaft. Thus, in comparison with the state of the art, friction can be advantageously reduced. As less unwanted mechanical friction is generated than in the state of the art, the quality of the active fixation electrode is improved and a smooth deployment of the active fixation electrode can be achieved.

For sake of completeness, it is noted that the expression "conductive driver shaft" refers to an electrically conductive driver shaft.

The implantable pacing and/or defibrillation lead described herein can be further improved according to various examples as shown below.

For example, the electrically conductive compression spring can be a closed coiled helical spring.

It allows advantageously reducing the size of the spring while facilitating the assembly of the lead as the closed coiled helical spring can be maintained around the tubular body without the need of additional retaining means. The successive coils of the spring provide multi-point contacts with the conductive driver shaft at the radial opening, therefore ensuring a redundancy of the electrical contacts.

For example, the electrically conductive compression spring can be an open coiled helical spring comprising two free-ends.

Hence, the solution for avoiding chatter noise can advantageously be implemented with different type of springs.

For example, at least a portion of the tubular body provided with the radial opening can have an outer radius greater than an inner radius of the electrically conductive compression spring at its rest state.

The radial dimension difference causes the spring to be forced against the conductive driver shaft by its restoring force and thus ensures the electrical contact.

For example, the radial opening can have an opening angle comprised between 100° and 250°, in particular between 150° and 210°, in a cross section of the tubular body perpendicular to a direction of extension of the lead.

The dimension of the radial opening is adapted to provide an opening allowing the spring to generate a restoring force sufficient to provide a stable contact against the conductive driver shaft.

For example, the radial opening can have a width along a direction of extension of the lead greater than the extension of the electrically conductive compression spring in that direction.

Hence, sufficient space is provided to reliably bring the spring into contact with the conductive driver shaft as the spring can freely retract onto the driver shaft when positioned in the radial opening.

For example, the inner diameter of the distal housing portion can be larger than the diameter of the electrically conductive compression spring at least partially radially arranged around the tubular body and its radial opening.

Hence, in the lead, because of their dimension's differences, the spring is not compressed by the distal housing portion, which would lead to unwanted larger friction.

For example, the tubular body can have at least one chamfered edge at the radial opening.

The presence of chamfered edge prevents damaging the spring, in comparison with sharp edge. Thereby, the robustness and the lifetime of the lead can be improved.

For example, the tubular body can comprise at least - a first portion provided with the radial opening and a portion of the tubular body contacting the distal housing portion, and - a second portion extending from a distal end of the tubular body to the first portion, and wherein a sealing means can be provided at the second portion to seal the first portion of the tubular body from the distal end of the lead.

Hence, the first portion of the tubular body, which comprises the spring, can be advantageously sealed against fluids like blood, being an ionic solution. It allows preventing surface and/or volume corrosion, in particular at the electrically conductive compression spring.

For example, the tubular body of the guiding member can be mechanically and electrically connected to the distal housing portion by means of a welding, and/or by means of a crimping means and/or a heat shrinked means arranged to maintain the tubular body in electrical and mechanical contact with the distal housing.

It further allows providing a lead easy to assemble, thereby improving the manufacturing efficiency

It further allows establishing a stable electrical connection between the tubular body of the guiding member and the distal housing portion.

The present disclosure also relates to a method for assembling an implantable pacing and/or defibrillation lead according to one of the above-mentioned examples.

Said method comprises the steps of: a) providing the electrically conductive compression spring around the tubular body of the guiding member at the radial opening such that a portion of the electrically conductive compression spring is partially deflected inside the lumen due to its restoring force, then b) inserting the conductive driver shaft through the lumen of the conductive guiding member thereby pushing at least partially the compression spring outside the lumen so as to provide an electrical connection between the electrically conductive compression spring and the conductive driver shaft, and then c) mechanically and electrically connecting the tubular body of the guiding member to the distal housing portion of the lead.

The method allows providing a simplified and reliable assembly process.

For example, step b) can further comprise providing a sealing means at the distal end of the tubular body to seal it from the distal end of the lead.

It allows sealing the tubular body from the distal end of the lead against fluids like blood, being an ionic solution. It thus allows preventing surface and/or volume corrosion, in particular at the electrically conductive compression spring.

For example, step c) can comprise welding the tubular body to the distal housing portion of the lead, in particular by laser welding.

It allows establishing a stable electrical connection between the tubular body of the guiding member and the distal housing portion.

The present invention provides a implantable pacing and/or defibrillation lead according to claim 1.

It is noted that the above-mentioned sleeve is a distal housing of portion of the lead.

The annular electrode of the lead body is superimposed to the sleeve. In other words, the annular electrode is arranged above the sleeve in a radial direction of the lead.

As a result, the rigid portion of the lead is advantageously compacted axially, i.e. along the longitudinal axis of the lead, which allows the length of the rigid portion to be reduced compared to the known leads.

In fact, two functions of the lead are superimposed because the annular electrode, which has an anode function for detection and/or stimulation is superimposed, i.e. arranged above, the sleeve comprising an electrode, which has a cathode function for detection and/or stimulation. The electrodes constitute so-called "rigid" portions of the lead. In the case of leads with active fixation, the sleeve comprises an electrode in the form of a retractable screw, which has a combined function of cathode for detection and/or stimulation and means of fixation to a tissue. The structure of the lead thus allows a plurality of lead functions to be arranged in a way that the length of the rigid portion of the lead can be reduced (i.e. along the longitudinal axis of the lead), thereby facilitating its implantation and placement within the body of a patient.

It also allows avoiding the presence of a more flexible portion between two rigid portions, as known from the leads according to the state of the art. Thus, the lead according to the present invention is more reliable and robust over time.

The implantable pacing and/or defibrillation lead for addressing the second object of the invention can be further improved according to various advantageous embodiments.

The electrically insulating sheath is arranged between the annular electrode and the sleeve in a radial direction of the lead. The annular electrode is thus superimposed to the sleeve, so that the annular electrode is arranged above the sleeve, but the annular electrode is not directly arranged on the sleeve.

The electrically insulating sleeve allows the isolation of the anode pole from the cathode pole. The use of a sheath on the sleeve has the advantage of being a compact structural solution.

In addition, the thermo-shrink formation avoids the presence of an air gap between the sleeve and the sheath and can easily be adapted to any shape.

The use of Teflon has the technical effect of improving the electrical insulation performance of the sheath because the efficiency of Teflon (tetra-fluoroethylene) in terms of electrical insulation is particularly satisfactory.

By using a metallic material for the sleeve, the wall thickness of the sleeve can be reduced in comparison to the thickness obtained by using a plastic material. Thus, either the diameter of the sleeve can be advantageously reduced, or the internal space of the sleeve can be enlarged, thus allowing the insertion of a fastening screw of larger diameter.

According to one embodiment, a proximal end of the sleeve can be accommodated within the distal end of the lead body and can comprise a shoulder.

The shoulder of the proximal end of the sleeve provides an axial stop on which the anode of the lead body can rest and thus mechanically hold the sleeve to the lead body.

According to one embodiment, the annular electrode can be attached to the sleeve by clamping or crimping.

Thus, the proximal end of the sleeve with the shoulder can be mechanically held to the lead body without the need for welding. This simplifies the assembly of the lead.

According to one embodiment, the annular electrode can be clamped or crimped in at least two distinct areas of the sleeve.

Thus, it is not necessary to crimp or clamp the annular electrode around its entire circumference to ensure the mechanical connection between the sleeve and the lead body. This further simplifies the assembly while ensuring a reliable mechanical connection over time. According to one embodiment, the implantable lead can further comprise a retaining ring welded or soldered to the annular electrode of the distal end of the lead body and arranged, along a radial direction of the lead, at least partially between the annular electrode and the sleeve, the retaining ring can further comprise at one end thereof a shoulder, said shoulder being arranged, along a longitudinal direction of the lead, between the annular electrode and the sleeve.

The use of a retaining ring offers an alternative way of securing the sleeve to the lead body.

According to one embodiment, the annular electrode can have a surface of at least 15 square millimeters.

This anode surface allows preserving sensing functions compatible with atrial and ventricular positioning.

According to one embodiment, the implantable lead can comprise an electrically insulating adhesive, in particular silicone adhesive or glue, between the distal end of the lead body and the proximal end of the sleeve accommodated in the distal end of the lead body.

Thus, the electrical isolation between the cathode and anode can be further improved, as the adhesive provides an additional sealing barrier.

In addition, the introduction of glue minimizes any relative movement between the sleeve and the distal end of the lead body.

The present invention also provides a method for assembling an implantable lead as defined in claim 8.

As a result, the rigid portion of the lead is advantageously compacted axially (i.e. along the longitudinal axis of the lead), which makes it possible to reduce the length of the rigid portion compared to known leads.

In fact, two functions of the lead are superimposed because the annular electrode, which has an anode function for detection and/or stimulation is superimposed to, i.e. arranged above, the sleeve comprising an electrode which has a cathode function for detection and/or stimulation. The electrodes constitute so-called "rigid" portions of the lead. In the case of leads with active fixation, the sleeve comprises an electrode in the form of a retractable screw, which has a combined function of cathode and means of fixation to a tissue. The structure of the lead thus allows a plurality of functions of the lead to be arranged in a way to reduce the length (i.e., along the longitudinal axis of the lead) of the rigid portion of the lead, thereby facilitating its implantation and placement within the body of a patient.

It allows also avoiding the presence of a more flexible portion between two rigid portions, as known from the lead of the state of the art. Thus, the lead according to the present invention is more reliable and robust over time.

The electrically insulating sleeve allows the insulation of the anode pole (i.e. the annular electrode) from the cathode pole (i.e. the electrode in the sleeve). The use of a sheath on the sleeve has the advantage of being a compact structural solution.

In addition, the thermo-shrink formation allows avoiding the presence of an air gap between the sleeve and the sheath and can easily be adapted to any shape.

According to one embodiment, the method can further comprise a step of clamping or crimping the annular electrode to the sleeve.

Thus, the proximal end of the sleeve with the shoulder can be mechanically held to the lead body without the need for welding. This simplifies the assembly of the lead.

According to one embodiment, step 2) of the method can further comprise: welding a retaining ring to the annular electrode of the lead body, and inserting the sleeve into the lead body such that the retaining ring is, in a radial direction of the lead, at least partially between the annular electrode and the sleeve, and such that a shoulder provided at one of the ends of the retaining ring is arranged, in a longitudinal direction of the lead, between the annular electrode and the sleeve.

The use of a retaining ring provides an alternative way of securing the sleeve to the lead body. According to one embodiment, the method can further comprise a step of introducing electrically insulating adhesive between the distal end of the lead body and the portion of the sleeve accommodated in the distal end of the lead body.

Thus, the electrical isolation between the cathode and anode can be further improved, as the adhesive provides an additional sealing barrier.

In addition, the introduction of glue allows minimizing any relative movement between the sleeve and the distal end of the lead body.

The present disclosure also relates to an implantable pacing and/or defibrillation lead, said lead extending from a proximal end to a distal end, wherein the proximal end of the lead is configured to be connected to an implantable medical device. The lead comprises: an active fixation electrode at the distal end which is mechanically and electrically coupled to a conductive driver shaft moveably arranged in a distal housing portion of the lead, wherein the active fixation electrode is movable from a retracted position in which the active fixation electrode is completely housed inside the distal housing portion, to an extended position, in which the active fixation electrode extends in a distal direction at least partially beyond the distal housing portion. The lead further comprises: - a guiding member arranged inside the distal housing portion and comprising a tubular body electrically connected with the distal housing portion, wherein the conductive driver shaft is slideably and rotatably arranged through a lumen extending through the tubular body, and - a sealing means for sealing the distal housing portion from the distal end of the lead. The sealing means comprises a sealing ring having at least two portions with respect to a central longitudinal axis of said sealing ring, a first portion of the sealing ring being arranged around the conductive driver shaft and a second portion of the sealing ring being arranged around the tubular body, the first portion of the sealing ring having an external diameter strictly less than an internal diameter of the distal housing portion, and the first portion of the sealing ring having an internal diameter strictly less than an external diameter of the conductive driver shaft, and the second portion of the sealing ring having an external diameter strictly more than the internal diameter of the distal housing portion.

Hence, the dimensions of the sealing ring of the sealing means allows avoiding fluidic communication from the distal end of the lead wherein blood can enter the distal housing portion. It thus allows at least partially sealing the distal housing portion, in particular wherein metallic elements may be housed, such as an electrically conductive spring for providing an electrical connection. The sealing means thus decrease the risk of surface and/or volume corrosion, in particular between a fluid and such an electrically conductive spring.

This is achieved thanks to the dimensions of the sealing ring, the first portion of which having dimensions allowing a rotation and a translation of the conductive driver shaft within the sealing ring while forming a seal at an interface between the conductive driver shaft and the sealing ring.

It is further achieved thanks to the dimensions of the second portion of the sealing ring forming a seal at an interface between the internal distal housing portion and the sealing ring.

As the first portion of the sealing ring have an external diameter strictly less than an internal diameter of the distal housing portion, there is no stress applied on the sealing ring by the distal housing portion at the first portion of the sealing ring. Hence, at the first portion, the sealing ring is only deformed, in particular elastically deformed, at the interface between the first portion and the conductive driver shaft.

For example, the percentage difference of diameter between the external diameter of the first portion of the sealing ring and the internal diameter of the distal housing portion can be comprised between 3 % and 30%, particular between 5% and 20%.

Hence, it allows reducing the friction between the sealing ring and the distal housing portion, so as to not hinder the rotation and the translation of the conductive driver shaft with respect to the sealing means at the first portion of the sealing ring. By avoiding friction, the active fixation electrode can be deployed more smoothly, without unwanted jerking motion of the conductive driver shaft.

Such range can render a fluidic communication possible at the interface between the distal housing portion and the first portion of the sealing ring. The sealing properties are however ensured at the interface between the conductive driver shaft and the first portion of the sealing means, and at the interface between the distal housing portion and the second portion of the sealing means.

For example, the percentage difference between the internal diameter of the first portion of the sealing ring and an external diameter of the conductive driver shaft be comprised between 0,05 % and 20%, in particular between 1% to 10%.

The above-mentioned range of percentage difference allows the conductive driver shaft to rotate and slide with respect to the sealing ring while preventing fluid communication at the interface between the conductive driver shaft and the first portion of the sealing ring.

For example, the percentage difference between the external diameter of the second portion of the sealing ring and the internal diameter of the distal housing portion can be comprised between 0,5 % and 20%, in particular between 1% and 10%.

The above-mentioned range of percentage difference allows forming a seal and preventing fluid communication at the interface between the distal housing portion and the second portion of the sealing ring.

For example, the internal diameter of the first portion of the sealing ring can be less than the internal diameter of the second portion of the sealing ring.

It allows providing a lead wherein the dimension of the first portion is adapted for receiving the conductive driver shaft while the dimension of the second portion is adapted for receiving the tubular body wherein the conductive driver shaft is slideably and rotatably arranged through the lumen of the tubular body.

For example, a distal end of the tubular body can be provided with an external radially protruding shoulder, said external radially protruding shoulder being form-fitted into a corresponding internal radial recess of the sealing ring of the sealing means.

The form-fitted connection allows locking the seal means to the tubular body, in particular a friction-locked connection.

For example, the internal radial recess of the sealing ring can be provided at a junction between the first portion and the second portion of the sealing ring.

Hence, a mechanically stable retention by the form-fitted connection can be obtained.

For example, the first portion of the sealing ring can have a rounded edge along its internal diameter.

It allows reducing the friction generated between the sealing ring and the distal housing portion, so as to not hinder the rotation and the translation of the conductive driver shaft with respect to the sealing means at the first portion of the sealing ring.

For example, the sealing means can be integrally formed in one-piece. Hence, the sealing means can be easily manufacturing and assembled to the lead, thereby reducing the assembly time and the assembly cost.

For example, the sealing means can be made of rubber, in particular of silicone, and can have a Shore hardness comprised between 50 to 65 Shore A.

The stress-strain behavior of a rubber sealing means can be linked to its Shore hardness. The selection of the Shore hardness in the range 50-65 Shore A allows ensuring that a sufficient compressive force is exerted on the flexible rubber sealing means in the distal housing portion. The Shore hardness of the rubber sealing means is thus adapted to provide the sealing properties in the arrangement of the sealing means inside the distal end housing.

The present disclosure further relates to an implantable pacing and/or defibrillation lead, said lead extending from a proximal end to a distal end, wherein the proximal end of the lead is configured to be connected to an implantable medical device, the lead comprising: an active fixation electrode at the distal end which is mechanically and electrically coupled to a conductive driver shaft moveably arranged in a distal housing portion of the lead, wherein the active fixation electrode is movable from a retracted position in which the active fixation electrode is completely housed inside the distal housing portion, to an extended position, in which the active fixation electrode extends in a distal direction at least partially beyond the distal housing portion, and an electrically conductive compression spring, further comprising: a guiding member arranged inside the distal housing portion and comprising a tubular body electrically connected with the distal housing portion, wherein the conductive driver shaft is slideably and rotatably arranged through a lumen extending through the tubular body, wherein the tubular body further comprises a radial opening in communication with the lumen, and wherein the electrically conductive compression spring is at least partially radially arranged around the tubular body and its radial opening, such that the electrically conductive compression spring is forced by its restoring force against the conductive driver shaft, and a sealing means for sealing the distal housing portion from the distal end of the lead, the sealing means comprising a sealing ring having at least two portions with respect to a central longitudinal axis of said sealing ring, a first portion of the sealing ring being arranged around the conductive driver shaft and a second portion of the sealing ring being arranged around the tubular body, the first portion of the sealing ring having an external diameter strictly less than an internal diameter of the distal housing portion, and the first portion of the sealing ring having an internal diameter strictly less than an external diameter of the conductive driver shaft, and the second portion of the sealing ring having an external diameter strictly more than the internal diameter of the distal housing portion.

Additional features and advantages of the present invention will be described with reference to the drawing. In the description, reference is made to the accompanying figure that is meant to illustrate the invention. It is understood that the scope of the present invention is defined by the appended claims.
**Figure 1** represents a cross-sectional view of a cardiac stimulation lead according to a first example.
Figure 2 represents a cross-sectional view of the cardiac pacing lead according to a second example.
**Figure 3A** depicts a cross-sectional view of a first step for the cardiac pacing lead according to the first example.
**Figure 3B** represents a cross-sectional view of a second step for assembling the cardiac pacing lead according to the first example.
**Figure 3C** depicts a cross-sectional view of a third step for assembling the cardiac pacing lead according to the first example.
**Figure 3D** represents a cross-sectional view of a fourth step for assembling the cardiac pacing lead according to the first example.
**Figure 3E** depicts a cross-sectional view of a fifth step for assembling the cardiac pacing lead according to the first example.
**Figure 3F** represents a schematic view of the fifth step for assembling the cardiac pacing lead according to the first example.
**Figure 3G** depicts a cross-sectional view of a sixth step for assembling the cardiac pacing lead according to the first example.
**Figure 4A** represents a cross-sectional view of a step for assembling the cardiac pacing lead according to the second example, prior to adhesive or glue deposition.
**Figure 4B** represents a cross-sectional view of a step for assembling the cardiac pacing lead according to the second example, after adhesive or glue deposition.
**Figure** 5 illustrates a cross-sectional view of a lead's distal end parallel to a direction of extension of the lead according to a third example.
**Figure 6A and Figure 6B** illustrate schematic views of a guiding member according to the third example.
Figure 7 illustrates a spring according to the third example.
**Figure 8** illustrates a cross-sectional view perpendicular to a direction of extension of the lead of the guiding member and the spring according to the third example.
**Figure 9A** illustrates a schematic view of an assembly comprising a conductive driver shaft, the guiding member and the spring according to the third example.
**Figure 9B** illustrates a cross-sectional view perpendicular to a direction of extension of the lead of Figure 9A.
Figure 10 illustrates a schematic view of an assembly comprising a helix screw, a sealing means, the conductive driver shaft, the guiding member and the spring according to the third example.
Figure 11 illustrates a cross-sectional view of a lead's distal end parallel to a direction of extension of a lead according to a fourth example.
**Figure 12** illustrates a cross-sectional view of a sealing means of the lead according to the first, the second and the third examples.
**Figure 13** illustrates a cross-sectional view of the distal end of lead according to the first, the second and the third examples. comprising the sealing means represented in Figure 12.

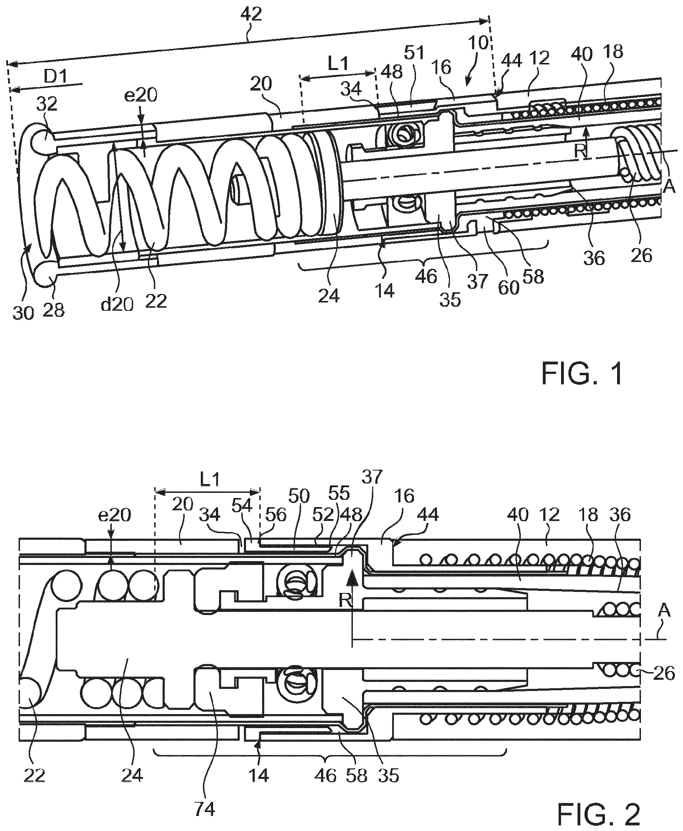

**Figure** 1 depicts a cross-sectional view of an implantable cardiac pacing lead 10 according to a first example. In one example, it may be a defibrillation lead.

The pacing lead 10 shown in Figure 1 is a retractable screw lead. In another example, it may be a passive fixation lead comprising barbs arranged radially around the lead body with a stimulation (pacing) electrode at the distal end.

The lead 10 comprises a substantially cylindrical lead body 12 that extends longitudinally along an axis A, which constitutes an axis of revolution.

The lead body 12 comprises a proximal end (not shown in Figure 1) configured for connection to an implantable medical device (not shown in Figure 1) and a distal end 14 that is opposite to said proximal end.

The distal end 14 of the lead body 12 comprises an annular electrode 16. The annular electrode 16 forms an anode of the lead 10. The annular electrode 16 and the lead body 12 have substantially the same diameter.

As shown in Figure 1, the distal end 14 of the lead body 12 is terminated by the annular electrode 16.

Between the distal end 14 and the proximal end of the lead 10, coiled electrical conductors 18 are housed within the lead body 12. In particular, the electrical conductors 18 allow the annular electrode 16, i.e., the anode, to be electrically connected to one pole of the implantable medical device connector (not shown in Figure 1).

The lead 10 further comprises a sleeve 20 in which a retractable fixation screw 22 is housed. The retractable attachment screw 22 forms a cathode of the lead 10. The retractable fixation screw 22 is a helical screw made of conductive material, connected through a metal tip 24, i.e. an electrically conductive driver shaft 24, to an internal conductor 26. The coiled conductor 26 provides electrical continuity between the retractable fixation screw 22 (which acts as a sensing and stimulation electrode) and a generator located at the proximal end (not shown in Figure 1) of the lead 10.

The sleeve 20 is substantially cylindrical in shape with the same diameter as the annular electrode 16 and the lead body 12.

The sleeve 20 comprises a distal end 28 provided with an opening 30 dimensioned such that the retractable fixation screw 22 is configured to be deployed along a direction D1 out of the sleeve 20 through said opening 30. The opening 30 in the distal end 28 is provided with an edge 32 around the entire circumference of the sleeve 20. The edge 32 is substantially rounded and therefore is not sharp or cutting. A sealing means 74 (visible in Fig. 1 but only annotated in Fig. 2) is provided in the sleeve 20 to at least partially seal the lead 10 from the distal end 28.

The sleeve 20 comprises, opposite to its distal end 28, a proximal end 34. At the proximal end 34, a mouthpiece or guiding member 35 of the lead 10 is bordered around its entire circumference by a shoulder 37.

The sleeve 20 is partially accommodated at its proximal end 34 within the distal end 14 of the lead body 12 such that the annular electrode 16 of the lead body 12 is superimposed on the sleeve 20, i.e., the annular electrode 16 is disposed above the sleeve 20 along a radial direction R of the lead.

As it will be described below, the annular electrode 16 is not disposed directly on the sleeve 20 due to the presence of an electrically insulating sheath (shown by the reference sign 48) disposed along the radial direction R of the lead between the annular electrode 16 and the sleeve 20.

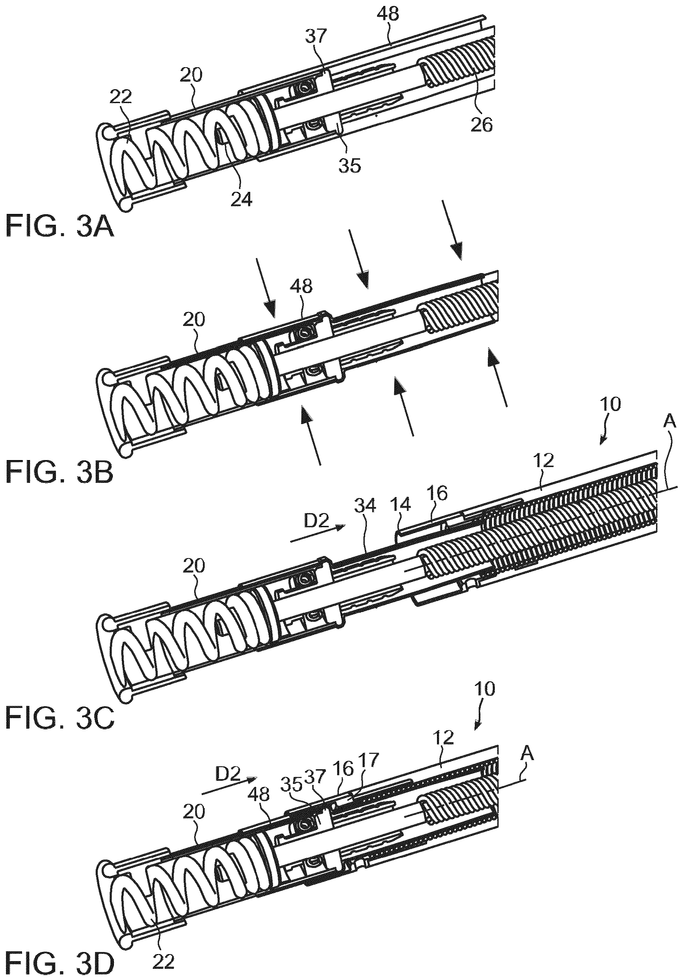

The structure and geometry of the sleeve 20 is further appreciated in the drawings of **Figures 3A-3C** described below, which illustrate the sleeve 20 outside the lead body 12.

In one example wherein the lead is a passive fixation lead, the stimulation electrode may have a substantially solid cylinder shape and may be at least partially housed within the sleeve 20 such that the stimulation electrode, like the retractable fixation screw 22, extends along the axis A. The distal end of the stimulation electrode can be a substantially smooth, flat or curved surface configured to contact tissue. Such a lead is then held in place by means of barbs arranged radially around the lead body.

The metal tip 24, i.e. the electrically conductive driver shaft 24, and the inner conductor 26 are housed in the lead body 12 and are arranged, in particular partially arranged, within the circumference 36 defined by spiral electrical conductors 18.

Thus, two functions of the lead 10 are superimposed along a radial direction R of the lead: a portion of the anode 16 (sensing/stimulation function) is superimposed with the sleeve 20 comprising the retractable screw 22 (fixation and sensing/stimulation function). As a result, the rigid portion 42 of the lead 20, i.e., the portion 42 that extends from the distal end 28 of the sleeve 20 to the junction 44 between the annular electrode 16 and the lead body 12, is advantageously compacted axially along the axis A (i.e., along the longitudinal axis of the lead 10), thereby allowing reducing the length of the rigid portion 42 as compared to that of known leads. This also makes it possible to avoid the presence of a flexible portion between two rigid portions, as proposed by some leads known from the state of the art.

The sleeve 20 is partially accommodated in the distal end 14 of the lead body 12 such that the distance L1 between the cathode 22 and the anode 16 is at least 3 millimeters. This distance L1 allows to preserve sensing functions that are compatible with an atrial and ventricular implantation of the lead 10.

In addition, the anode 16 preferably has a surface area of at least 15 square millimeters to preserve sensing and pacing functions compatible with an atrial and ventricular implantation of the lead 10.

The sleeve 20 is made of a metallic material. The use of a metallic material for the sleeve 20 allows the thickness "e20" of the wall of the sleeve 20 to be reduced in comparison to the thickness involved by the use of a plastic material. Thus, either the diameter d20 of the sleeve 20 can be advantageously reduced, or the internal volume of the sleeve can be enlarged, thereby allowing the insertion of a fixation screw 22 of larger diameter.

The conductive elements of the sleeve 20, i.e., the retractable fixation screw 22, the electrically conductive driver shaft 24, and the inner conductor 26, are insulated from the conductive elements (i.e. the coiled electrical conductors 18) connected to the annular electrode 16 of the lead body 12 by a cylindrical tube 40 made of an electrically insulating material.

In addition, in order to electrically isolate the cathode (i.e. the retractable fixation screw 22) from the anode (i.e. the annular electrode 16) at the overlap area 46 of the sleeve 20 at the distal end 14 of the lead body 12, an electrically insulating sheath 48 is disposed on the sleeve 20. The electrically insulating sheath 48 is disposed between the annular electrode 16 and the sleeve 20 along the radial direction of the lead 10. The electrically insulating sheath 48 may be a Teflon sheath heat shrunk onto the sleeve 20 at the overlap area 46.

The electrically insulating sleeve 48 allows the isolation of the pole constituted by the anode 16 from the pole constituted by the cathode 22, in a radial direction R of the lead body 12. The use of a sheath 48 arranged on the sleeve 20 has the advantage of being a structurally compact solution. Indeed, the sheath 48 can be arranged in the residual space between the sleeve 20 and the lead body 12 superimposed on the sleeve 20, which is typically less than 50 microns.

In addition, the heat shrink formation eliminates the need for an air gap between the sleeve 20 and the sheath 48 and allows for easy adaptation to any shape.

Moreover, the efficiency of Teflon (tetra-fluoroethylene) in terms of electrical insulation is particularly satisfactory.

However, the use of a Teflon sheath 48 does not allow the use of known conventional adhesives such as silicone or polyurethane to ensure the mechanical connection between the sleeve 20 and the lead body 12, as Teflon constitutes a non-stick (i.e. non-adhesive) coating. Therefore, as shown in Figure 1, the annular electrode 16 is deformed, by clamping or crimping, to form a recess 51. This recess 51 abuts on the shoulder 37 of the mouthpiece 35 of the sleeve 20. In other words, the shoulder 37 provides an axial stop for the anode 16, which mechanically holds the sleeve 20 to the lead body 12.

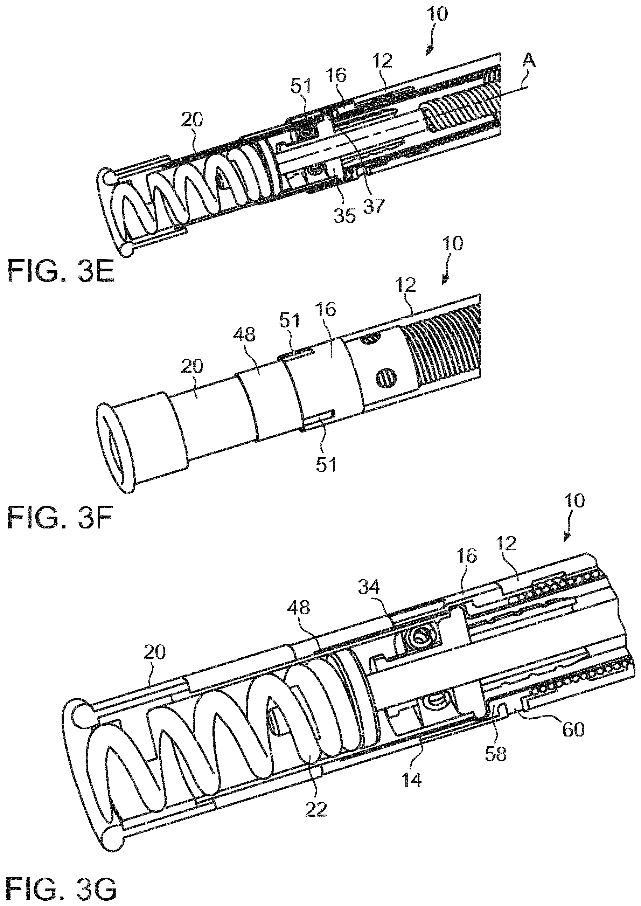

As illustrated in Figure 3F, which will be further described below, the anode 16 may comprise at least two recesses 51, preferably three, distributed distinctly from one another around the circumference of the annular electrode 16. This eliminates the need to crimp the entire circumference of the anode 16, thereby preserving the underlying electrically insulating sheath 48, in particular the Teflon coating.

Figure 2 illustrates a second example for realizing the mechanical connection between the sleeve 20 and the lead body 12.

The elements with the same numerical references used for the description in Figure 1 refer to the same elements and will not be described again in detail.

In the second example, the anode 16 is not plastically deformed.

As illustrated in Figure 2, the lead 10 further comprises a retaining ring 50. The retaining ring 50 is welded to the annular electrode 16. In particular, the retaining ring 50 is welded to the inner wall 52 of the annular electrode 16. The retaining ring 50 comprises at its distal end 54 a shoulder 56.

According to the second example, the retaining ring 50 is arranged, in a radial direction R of the lead, partially between the annular electrode 16 and the sleeve 20. Because of the presence of the shoulder 56, the retaining ring 50 is arranged in a longitudinal direction of the lead 10, i.e., along the axis A, between the annular electrode 16 and the sleeve 20. As shown in Figure 2, the shoulder 56 abuts the distal end 14 of the lead body 12 in a direction parallel to the axis A.

The use of a retaining ring 50 provides an alternative to the first embodiment for securing the sleeve 20 relative to the lead body 12.

In both the first example (shown in Figure 1) and the second example (shown in Figure 2), electrically insulating adhesive 58, in particular silicone adhesive, may be introduced between the distal end 14 of the lead body 12 and the proximal end 34 of the sleeve 20 accommodated in the distal end 14 of the lead body 12 (i.e., the portion of the sleeve 20 covered by the electrically insulating sheath 48). This electrically insulating adhesive 58 may be introduced via a through-hole 60 provided in the lead body 12 at the annular electrode 16 (see Figure 1).

Thus, electrical isolation between cathode 22 and anode 16 can be further improved, as the adhesive 58 provides an additional sealing barrier.

In addition, the introduction of adhesive 58 allows minimizing any residual relative movement between the sleeve 20 and the distal end 14 of the lead body 12.

The electrically insulating adhesive 58 provides an adhesive seal 58 that ensures an axial locking by compensating the assembly clearances. In addition, the adhesive seal 58 provides a flexible stop between the proximal end 55 (opposite the distal end 54 along the axis A) of the retaining ring 50 and the shoulder 37 to prevent a progressive piercing of the insulating sleeve 48.

As in the first example, the sealing means 74 is provided in the sleeve 20 to at least partially seal the lead 10 from the distal end 28.

A method for assembling a stimulation lead 10 according to the first example is described below with reference to **Figures** 3A-3G.

The elements with the same numerical references used for the description in Figure 1 refer to the same elements and will not be described again in detail.

**Figure 3A** shows a cross-sectional view of the sleeve 20 in which the retractable fixation screw 22 is housed. The electrically insulating sleeve 48 is positioned around the sleeve 20 so as to at least partially cover the retractable fixation screw 22, the electrically conductive drive shaft 24, the mouthpiece 35 and the shoulder 37, and the coiled electrical conductors 26.

**Figure 3B** depicts a next step in the method in which the electrically insulating sheath 48 is heat shrunk to the sleeve 20. The electrically insulating sleeve 48 is thereby deformed to conform to the shape of the outer circumference of the sleeve 20.

**Figure 3C** depicts a next step of the method. The proximal end 34 of the sleeve 20, opposite to the distal end 28 along the axis A, is provided with the electrically insulating thermoformed sheath 48 and is accommodated in the lead body 12 by the distal end 14 of the lead body 12 along an insertion direction D2 parallel to the longitudinal axis A of the lead 10. As described with respect to Figure 1, the distal end 14 of the lead body 12 comprises the annular electrode 16.

**Figure 3D** depicts a next step in the method in which the sleeve 20 has been partially accommodated along the direction of insertion D2 into the lead body 12 until the shoulder 37 of the mouthpiece 35 abuts an inner wall 17 of the annular electrode 16. Electrical insulation between the annular electrode 16 and the retractable fixation screw 22 is provided by the electrically insulating thermoformed sheath 48.

**Figure 3E** depicts a cross-sectional view of a subsequent method step in which the annular electrode 16 is plastically deformed by clamping or crimping to form elongated recesses 50 that extend in a direction parallel to the axis A of the lead 10.

**Figure 3F** illustrates the lead 10 in a three-dimensional schematic view. Two recesses 51 are visible around the circumference of the annular electrode 16 in Figure 3F.

As explained with reference to Figure 1, each recess 51 abuts against shoulder 37 of the mouthpiece 35 of the sleeve 20, i.e., shoulder 37 provides an axial stop for the anode 16, which mechanically holds the sleeve 20 to the lead body 12.

**Figure 3G****,** which corresponds to Figure 1, depicts a subsequent method step in which electrically insulating adhesive 58, in particular silicone adhesive, is introduced through the through-hole 60 of the annular electrode 16.

This insulating adhesive 58 disposed between the distal end 14 of the lead body 12 and the proximal end 34 of the sleeve 20 accommodated in the distal end 14 of the lead body 12 (i.e., the portion of the sleeve 20 covered by the electrically insulating sheath 48) improves the electrical isolation between the cathode 22 and the anode 16 by providing an additional sealing barrier.

In addition, the introduction of adhesive 58 allows minimizing any residual relative movement between the sleeve 20 and the distal end 14 of the lead body 12.

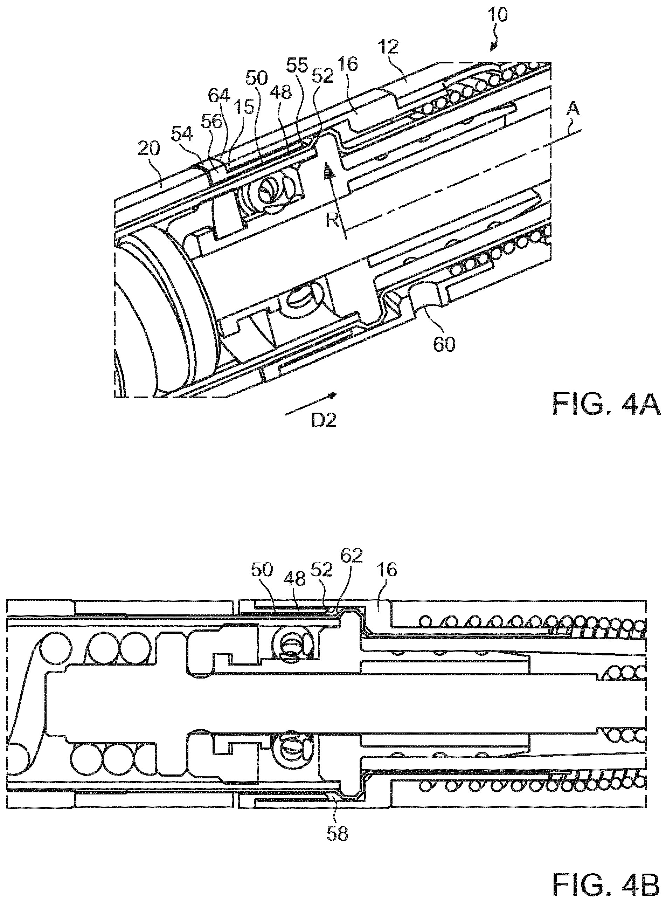

Steps of a method for assembling a stimulation lead 10 according to the second example are described below with reference to Figures 4A and 4B.

The elements with the same numerical references used for the description of Figures 1 and 2 refer to the same elements and will not be described again in detail.

**Figure 4A** shows a cross-sectional view of the sleeve 20 in which the retaining ring 50 has been welded at the interface 64, between the distal end 15 of the annular electrode 16 of the lead body 12 and the shoulder 56. The interface 64 thus extends along the radial direction R.

In this step of the method shown in Figure 4A, the sleeve 20 has further been inserted into the lead body 12 along an insertion direction D2 parallel to the axis A of the lead 10. As a result, the retaining ring 50 is, along a radial direction R of the lead 60, at least partially disposed between the annular electrode 16 and the sheath 48 covering the sleeve 20. Furthermore, the shoulder 56 at the distal end 54 of the retaining ring 50 is disposed, along the longitudinal direction A of the lead, between the annular electrode 16 and the sleeve 20.

**Figure 4B****,** which corresponds to Figure 2, depicts a subsequent method step in which electrically insulating adhesive 58, in particular silicone adhesive, is introduced through the through-hole 60 (only visible in Figure 4A) of the annular electrode 16.

This insulating adhesive 58 is thus disposed in the residual space 62 between the inner wall 52 of the annular electrode 16, the retaining ring 50 and the electrically insulating thermoformed sheath 48.

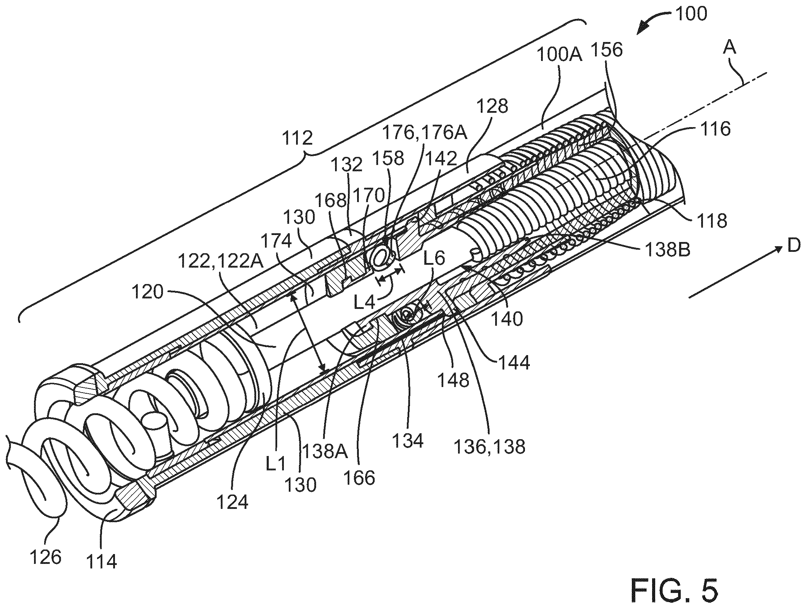

In the following, the avoidance of chatter noise between the retractable fixation screw 22, i.e. the helix electrode 22, and the sleeve 20, which constitutes a metallic distal housing portion of the lead, is explained with respect to the description of Figures 5 to 11.

**Figure 5** illustrates schematically an implantable pacing and/or defibrillation lead 100 according to a third example.

In the following, the implantable pacing and/or defibrillation lead 100 is also referred as "the lead 100".

In particular, Figure 5 shows a cross-sectional view parallel to a direction of extension D of the lead 100 of a portion 112 of the lead 100. The direction of extension D of the lead 100 is parallel to a central longitudinal axis A of the lead 100.

As the lead 10 in the first and the second examples, the lead 100 is provided with an elongated lead body 100A extending along the central longitudinal axis A from a proximal end (not represented in Figure 5) to a distal end 114.

The proximal end (not represented in Figure 5) of the lead 100 is configured to be connected to an implantable medical device (not represented in Figure 5) in a manner known per se.

At least one coil conductor 116 extends from the proximal end of the lead 100 (said proximal end is not visible in the partial view of the lead 100 represented in Figure 5), through a lumen 118 of the lead 100, toward the distal end 114 of the lead 100. The coil conductor 116 is mechanically and electrically connected to a conductive driver shaft 120 moveably arranged along the central longitudinal axis A in a distal housing portion 122 of the lead 100. Although it is not visible in the view of Figure 5, the coil conductor 116 is further mechanically and electrically connected to an implantable medical device at the proximal end of the lead 100.

It is noted that the distal housing portion 122 is a distinct piece from the elongated lead body 100A.

As in the first and the second examples, a distal end 124 of the conductive driver shaft 120 is provided with an active fixation electrode 126, in particular a helix electrode 126. The active fixation electrode 126 is mechanically and electrically coupled to the conductive driver shaft 120 so as to form a retractable and extendable mechanism. Hence, the active fixation electrode 126 is movable along the central longitudinal axis A from a retracted position in which the active fixation electrode 126 is completely housed inside the distal housing portion 122, to an extended position, as shown in Figure 5, in which the active fixation electrode 126 extends in a distal direction at least partially beyond the distal end 114 of the distal housing portion 122 of the lead 100. The active fixation electrode 126 is electrically connected to the coil conductor 116 by means of the conductive driver shaft 120.

As the sleeve 20 in the first and the second examples, in the third example the distal housing portion 122 of the lead 100 is provided with a cylindrical metallic body 122A of diameter L1. The use of metal, instead of plastic material, for the distal housing portion 122 allows reducing the dimension of the lead 100, in particular the longitudinal dimension of the lead 100 as explained above in reference to Figures 1 to 4B. Indeed, it allows advantageously radially superposing a ring electrode 128 (i.e. an annular electrode) provided around the elongated lead body 100A to the retractable and extendable mechanism formed by the conductive driver shaft 120 and the active fixation electrode 126 at the distal housing portion 122.

The distal housing portion 122 is partially covered by an outer insulated sheath 130 preferably made of silicone rubber or polyurethane.

As in the second example, the ring electrode 128 according to the third example, i.e. the anode of the lead 100, is not plastically deformed, e.g. by crimping.

Moreover, a retaining ring 132 is provided between the outer insulated sheath 130 and the ring electrode 128. However, the retaining ring 132 according to the third example has a different shape and arrangement than the retaining ring 150 of the second example, in that a portion of the retaining ring 132 extends under the outer insulated sheath 130.

An electrically insulated sheath 134, in particular a heat-shrinkable sleeve 134, more in particular a Teflon sheath 134, is provided between the distal housing portion 122, the outer insulated sheath 130, the retaining ring 132 and the ring electrode 128 for electrically isolating the ring electrode 128 from the active fixation electrode 126.

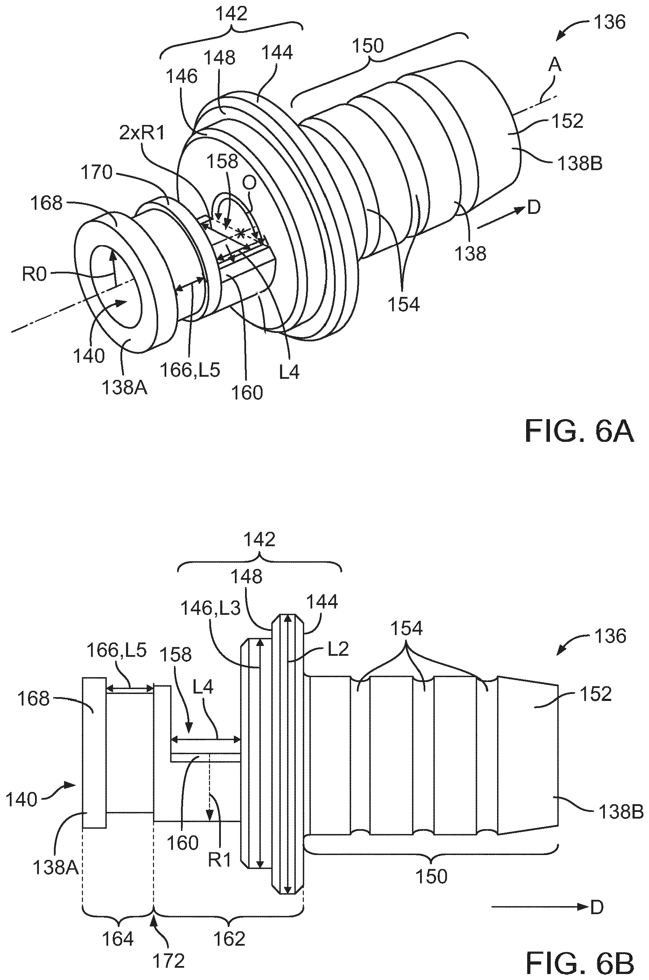

The lead 100 further comprises a guiding member 136 arranged inside the distal housing portion 122. Views of the guiding member 136 are further shown in Figures 6A and 6B to which reference is made in the following.

It is noted that the guiding member 136 is represented in Figures 1 to 4B by the mouthpiece 35. In fact, the mouthpiece 35 according to the first and second examples is the same as the guiding member 136 according to the third example.

Therefore, the description thereafter of the guiding member 136 also applies to the mouthpiece 35 according to the first and second examples.

The guiding member 136 comprises a tubular body 138 wherein a central lumen 140 of radius R0 extends therethrough from a distal end 138A to a proximal end 138B of the tubular body 138. As shown in Figure 5, the conductive driver shaft 120 is slideably arranged through the lumen 140 of the tubular body 138.

The tubular body 138 is electrically connected with the distal housing portion 122, in particular by means of an annular shoulder member 142 comprising a first annular shoulder 144 of diameter L2 and a second annular shoulder 146 of diameter L3, as indicated in Figure 6B. The first annular shoulder 144 and the second annular shoulder 146 extend radially from the tubular body 138. The diameter L2 is greater than the diameter L3. As a result, a step 148 is formed between the first annular shoulder 144 and the second annular shoulder 146. Moreover, the diameter L3 is substantially equal to the diameter L1 of the distal housing portion 122. As shown in Figure 5, the tubular body 138 is thus adapted for receiving the distal housing portion 122 at the step 148 of the annular shoulder member 142. The distal housing 122 thus abuts, along the central longitudinal axis A, on the first annular shoulder 144, as shown in Figure 5. The tubular body 138 can be welded to the distal housing portion 122, in particular at the step 148, preferentially by laser welding. The laser weld allows providing long term mechanical strength and electrical continuity between the tubular body 138 and the distal housing portion 122.

Between the annular shoulder member 142 and the proximal end 138B, the tubular body 138 comprises an elongated guiding portion 150. As shown in Figure 5, the central lumen 140 at the elongated guiding portion 150 is adapted for partially receiving the conductive driver shaft 120 and the coil conductor 116. The outer wall 152 of the elongated guiding portion 150 is provided with a plurality of annular grooves 154 (visible in Figures 6A and 6B) for realizing a frictional fitting with a seal member 156 of the lead 100, as shown in Figure 5. Hence, a mechanical connection is provided between the seal member 156 and the elongated guiding portion 150.

As best shown in Figures 6A and 6B, between the annular shoulder member 142 and the distal end 138A, the tubular body 138 comprises a radial opening 158 in communication with the lumen 140. In the example of Figure 6A, the radial opening 158 has an opening angle O of approximatively 180° in a cross section of the tubular body 138 perpendicular to a direction of extension D of the lead 100, which is indicated by the arrow D in Figures 6A and 6B. In a variant, the opening angle O can be comprised between 100° and 250°, in particular between 150° and 210°. The radial opening 158 has a width L4 along the direction of extension D of the lead 100.

As shown in Figures 6A and 6B, the tubular body 138 has at least one chamfered edge 160 of length T1 at the radial opening 138. The chamfered edge 160 extends along the direction of extension D of the lead 100.

As illustrated in Figure 6B, the radial opening 158 and the annular shoulder member 142 define a first portion 162 of the tubular body 138. In the first portion 162, at the radial opening 158, the tubular body 138 has an outer radius R1, as indicated in Figures 6A and 6B. As it can be understood from Figure 6A, the outer radius R1 corresponds to the sum of the radius R0 of the lumen 140 and a lateral wall thickness of the tubular body 138. It is noted that in Figure 6A, the arrow indicated by "2xR1" shows the diameter of the tubular body 138, which corresponds to two twice the radius R1.

The tubular body 138 comprises a second portion 164 extending from the distal end 138A of the tubular body 138 to the first portion 162. The second portion 164 is provided with a groove 166 of width L5 along the direction of extension D of the lead 100. The second portion 164 is provided with an external radially protruding shoulder 168 at the distal end 138A. An annular shoulder 170 is formed at the junction 172 between the first portion 162 and the second portion 164.

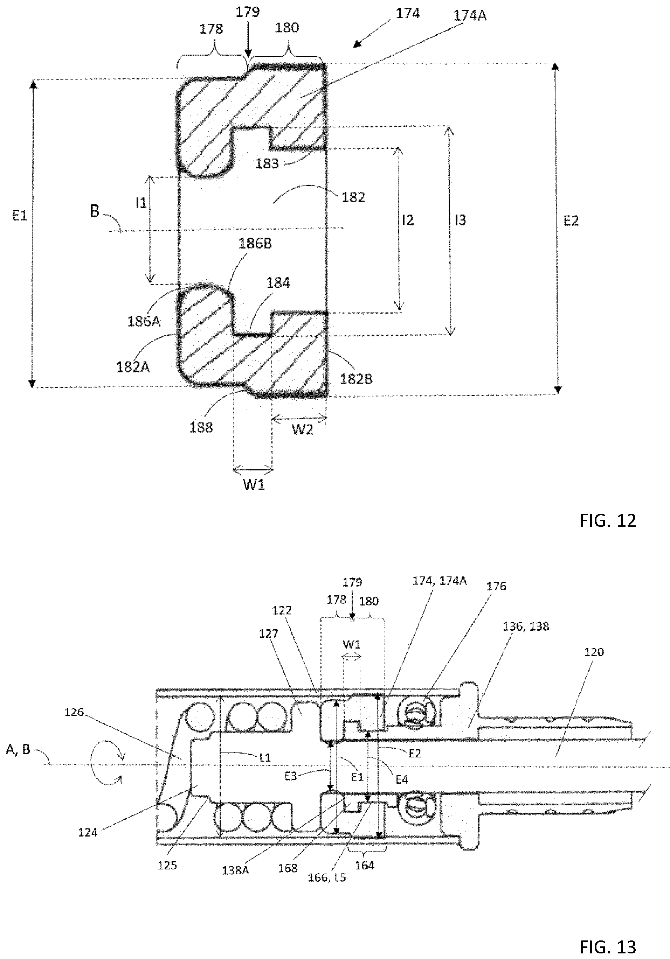

As shown in Figure 5, a sealing means 174 is provided at the second portion 164 to seal the first portion 162 of the tubular body 138 from the distal end 114 of the lead 100. The sealing means 174 is form-fitted to the second portion 162, in particular by means of the groove 166 delimited between the external radially protruding shoulder 168 and the annular shoulder 170. The sealing means 174 realizes a frictional fitting with the conductive driver shaft 120 and the distal housing portion 122, as can be seen in the cross-sectional view of Figure 5. The sealing means 174 is further described in reference to Figures 12 and 13.

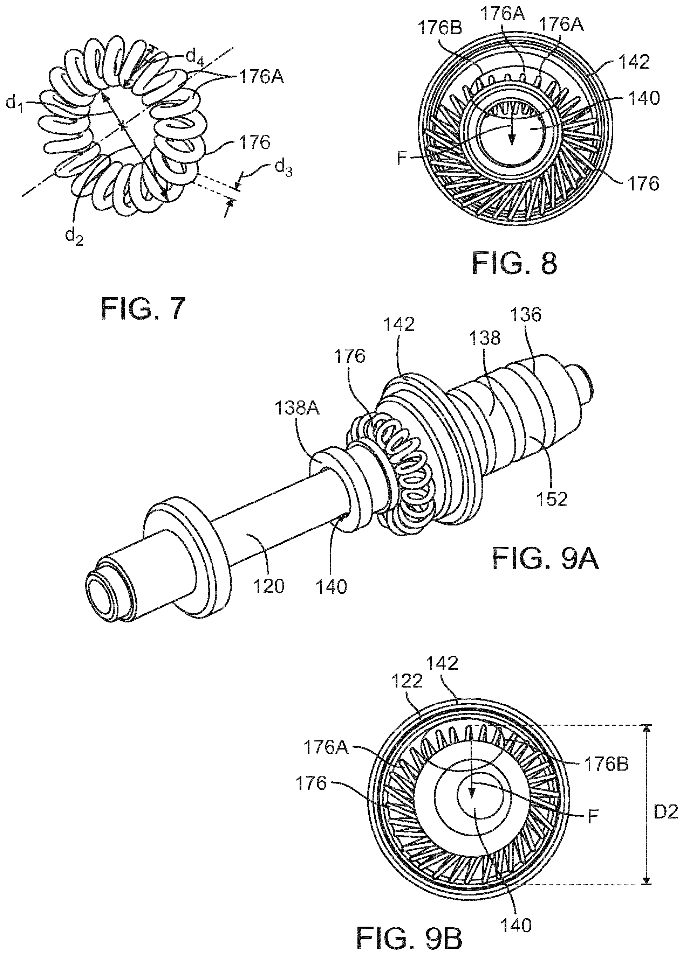

The lead 100 further comprises an electrically conductive compression spring 176. The spring 176 is preferentially a closed monowire spring 176, as illustrated in **Figure** 7. The spring 176 is thus a closed helical compression spring 176 comprising a plurality of coils 176A along its length.

In a variant, the spring 176 is an open spring comprising two free-ends, such as an open helical compression spring. In particular, the spring 176 can be a mono-wire (i.e. a single wire) open spring.

The spring 176, when it is not arranged within the lead 100 as illustrated in Figure 7, has the following dimensions are indicated in the Table 1 below. It is understood that at the rest state of the spring 176, the spring 176 is not assembled or mounted to any elements of the lead 100. Figure 7 represents the spring 176 at its rest state.

**Table 1.**

| **Feature of the spring 176** | **Dimension of the spring 176 at its rest state** |
|---|---|
| Inner radius d1 | 0,4mm to 1mm |
| Outer radius d2 | 1,5mm to 4mm, in particular 1,5mm to 2,5mm |
| Mono-wire thickness d3 | 0,1mm to 0,5mm |
| Coil's diameter d4 | 1mm to 2mm |

The inner radius d1 of the spring 176 at its rest state is less than the outer radius R1 of the first portion 162 of the tubular body 138 at the radial opening 158.

The spring 176 is preferentially made of platinum iridium, which is a biocompatible material exhibiting satisfying resistance to corrosion adapted for the application of the implantable lead 100. It is noted that the guiding member 136 is also preferentially made of platinum iridium, in particular for avoiding galvanic corrosion.

The spring 176 is at least partially radially arranged around the tubular body 138 and its radial opening 158, such that the spring 176 is forced by its restoring force F against the conductive driver shaft 120. As a result, the coils 176A that are partially deflected within the radial opening 158 provide multiple contact points with the conductive driver shaft 120. Thereby, a continuous electrical contact between the active fixation electrode 126 and the distal housing portion 122 is realized by means of the spring 176, which allows avoiding the occurrence of chatter noise.

As the coils 176A of the spring 176 only contact the conductive driver shaft 120 at the radial opening 158, the additional friction generation caused by the coils 176A in contact with the conductive driver shaft 120 is negligible, in particular with respect to the quality of the deployment and the motion of the active fixation electrode 126.

In the followings, a method for assembling the implantable pacing and/or defibrillation lead 100 according to the third example is described.

At a first step, the spring 176, which is illustrated in Figure 7, is provided around the tubular body 138 of the lead 100 at the radial opening 158 such that a portion 176B (indicated by a circle 176B on Figure 8) of the spring 176 is partially deflected inside the lumen 140 due to its restoring force F. The restoring force F of the spring 176 corresponds to the force F, which acts to bring the spring 176, in particular the portion 176B of the spring 176, to its equilibrium position. The equilibrium position of the spring 176 is the position wherein its potential energy is minimum. As the spring 176 exerts radial forces, according to the principle of minimum potential energy for a spring, the 176B portion of the spring 176 enters, in particular is clipped to, the radial opening 158, as shown in Figure 8, to reach a lower potential energy position.

In all variants of the present invention, the dimension of the opening angle O of the radial opening 158 is adapted to provide a sufficient opening for allowing a deflection of the spring 176 to a position of lower potential energy.

It is noted that, as indicated in Figure 5, the radial opening 158 has a width L4 along the direction of extension D of the lead 100 greater than the extension L6 of the spring 176 in the direction of extension D. It allows providing sufficient space for the deflection of the spring 176 towards a position of lower potential energy in the radial opening 158.

In comparison with the lead known from the state of the art, the assembly is facilitated because the spring 176 can be simply slide and clipped to the tubular member 138 at the radial opening 158, and thus does not require to be welded. Automated manufacturing is thus advantageously rendered possible.

The presence of the chamfered edges 160 allow avoiding damaging the spring 176 at the radial opening 158.

Then, at a second step illustrated by Figures 9A and 9B, the conductive driver shaft 120 is inserted via the distal end 138A through the lumen 140 of the conductive guiding member 136 thereby pushing at least partially the spring 176 outside the lumen 140 so as to provide an electrical connection between the spring 176, in particular between the coils 176A of the portion 176B, and the conductive driver shaft 120, as best shown in the cross-sectional view of Figure 9B.

The plurality of coils 176A of the portion 176B of the spring 176 provide redundancy for the contacts to ensure a reliable connection. It is further noted that the ratio of the number of coils, or spirals, of the spring 176 with respect to the length of the wire forming the spring 176 is selected so as to provide sufficient deflection to the portion 176B combined with a low bearing force on the friction surface of the conduction shaft 20. It therefore allows providing a limited parasitic friction torque.

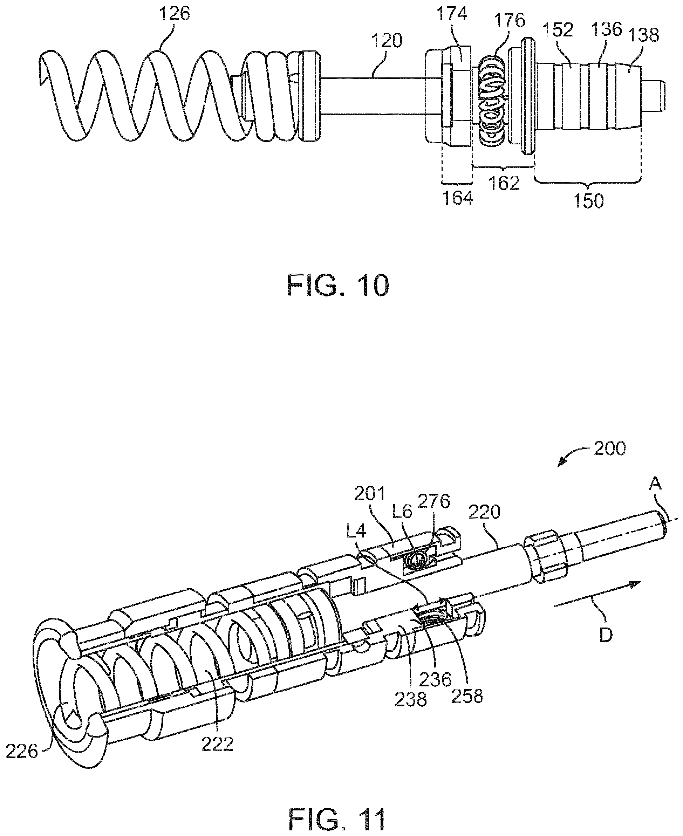

At a third step, illustrated in Figure 10, the active fixation electrode 126 is mechanically and electrically connected by laser welding to the distal end 124 of the conductive driver shaft 120. Moreover, the sealing means 174 is provided at the second portion 164 of the tubular body 138. It is noted that the sealing means 174 is illustrated in transparency in Figure 10.

It is noted that, in an alternative, the active fixation electrode 126 can be mechanically and electrically connected by laser welding to the distal end 124 of the conductive driver shaft 120 before the insertion of the conductive driver shaft 120 in the lumen 140 of the conductive guiding member 360.

In both alternatives, an assembly comprising the active fixation electrode 126, the conductive driver shaft 120, the sealing means 174, the spring 176 and the conductive guiding member 136, as illustrated in Figure 10, is obtained.

Then, the distal housing portion 122 is provided around the assembly illustrated in Figure 10. The distal housing portion 122 is mechanically and electrically connected to the tubular member 138 of the conductive guiding member 136 by laser welding. An electrically insulated sheath 34 can be heat-shrinked (i.e. heat-retracted) at least partially around the resulting assembly, as shown in Figure 5.

The resulting assembly of the conductive driver shaft 120, the conductive guiding member 136 and the electrically conductive compression spring 176 is arranged at the distal end 114 of the lead 100 as shown in Figure 5.

**Figure 11** illustrates a fourth example of an implantable pacing and/or defibrillation lead 200. Reference signs with the same tens unit than the reference signs already described and illustrated in Figures 5 to 10 will not be described in detail again but reference is made to their description above, because they relate to same functional and/or structural elements.

As explained in reference to the third example, the continuous electrical contact between the active fixation electrode 226 and the distal housing portion 222 for preventing chatter noise is realized by means of the electrically conductive compression spring 276 being at least partially radially arranged around the tubular body 238 of the guiding member 236 and its radial opening 258, such that the electrically conductive compression spring 276 is forced by its restoring force against the conductive driver shaft 220.

In the third and the fourth examples, in the assembly state as illustrated in Figures 5 and 11, the radial opening 158, 258 has a width L4 along the direction of extension D of the lead 100, 200 greater than the extension L6 of the electrically conductive compression spring 176, 276 in that direction. As already explained above, it allows providing sufficient space for the deflection of the spring 176, 276 towards a position of lower potential energy in the radial opening 158, 258.

In the fourth example, the spring 276 is enclosed by an annular spring cover 201, instead of the distal housing portion 222 (as in the third example).

However, in the assembly state, the inner diameter L1 of the distal housing portion 122 (shown in Figure 5), or of the annular spring cover 201, is larger than the outer diameter D2 of the spring 176, 276. It is noted that the reference D2 refers to the outer diameter of the spring 176, 276 in the assembly state, as illustrated in Figure 5 and 9B, while the reference d2 refers to the outer radius of the non-assembled spring 176, 276 in its rest state, as shown in Figure 7.

The electrical connection is thus not provided by a radial compression of the spring 176, 276 toward the conductive driver shaft 120, 220 applied by means of a recess or cage radially compressing the spring 176, 276, as in the lead known from the state of the art.

The purpose of the enclosure provided by the distal housing portion 122 to the spring 176 in the third example, or by the annular spring cover 201 to the spring 276 in the fourth example, is to protect the spring 176, 276 from the external environment, in particular from damage like corrosion, or loss.

In Figure 11, a sealing means 274 is provided inside the distal housing portion 222 to seal an interface between the conductive driver shaft 220 and the distal housing portion 222.

In the following, the sealing means 174 of the lead 100 according to the third example is further described in reference to **Figures 12 and 13****.** The description herebelow related to the sealing means 174 also applies to the sealing means 74 of the lead 10 according to the first example and the second example (as shown in Figure 2).

As best shown in **Figure 12****,** the sealing means 174 comprises a sealing ring 174A of central longitudinal axis B having at least two portions 178, 180 disposed along the central longitudinal axis B of said sealing ring 174A.

The sealing means 174 is made of rubber, in particular of silicone, and is integrally formed in one-piece. The sealing means has a Shore hardness comprised between 50 to 65 Shore A.

Figure 12 illustrates a cut-view perpendicular to the central longitudinal axis B of the sealing ring 174A. The central longitudinal axis B of the sealing ring 174A corresponds to the axis of revolution of the sealing ring 174A.

The sealing ring 174A has a lumen 182 defined by an inner wall 183 of the sealing ring 174A. The lumen 182 extends along the central longitudinal axis B from a distal end 182A to a proximal end 182B of the sealing ring 174A.

In the following, the expression "internal diameter" is to be understood as an inner diameter, i.e. a diameter of the lumen 182 defined by the inner wall 183.

The first portion 178 of the sealing ring 174A has an internal diameter I1. The second portion 180 of the sealing ring 174A has an internal diameter I2.

The internal diameter I1 of the first portion 178 of the sealing ring 174A is less than the internal diameter I2 of the second portion 180 of the sealing ring 174A.

The lumen 182 of the sealing ring 174A is provided with an internal radial recess 184 of width W1 (along the central longitudinal axis B) at a junction 179 between the first portion 178 and the second portion 180 of the sealing ring 174A. At the junction 179, the internal radial recess 184 has an internal diameter I3.

The internal diameter I2 of the second portion 180 of the sealing ring 174A is less than the internal diameter I3 of the internal radial recess 184 of the sealing ring 174A.

In the following, the expression "external diameter" is to be understood as an outer diameter of the sealing ring 174A.

The first portion 178 of the sealing ring 174A has an external diameter E1. The second portion 180 of the sealing ring 174A has an external diameter E2.

The external diameter E1 of the first portion 178 of the sealing ring 174A is less than the external diameter E2 of the second portion 180 of the sealing ring 174A. Hence, as shown in Figure 12, the first portion 178 and the second portion 180 are connected by a slope 188 at the junction 179.

The inner wall 183 at the first portion 178 is provided with a rounded edge 186A at the distal end 182A. The inner wall 183 at the first portion 178 is further provided with a rounded edge 186B between the first portion 178 and the junction 179.

The second portion 180 has a width W2 along the central longitudinal axis B.

**Figure 13** illustrates a cut-view perpendicular to the central longitudinal axis A of the lead 100. In particular, Figure 13 represents a partial view of Figure 5. Hence, the elements with the same numerical references used for the description in Figure 5 refer to the same elements and will not be described again in detail.

The conductive driver shaft 120 comprises a shoulder 125 at its distal end 124. The conductive driver shaft 120 further comprises an external radially protruding shoulder 127. The external radially protruding shoulder 127 is longitudinally arranged between the active fixation electrode 126 and the sealing means 174.

As already mentioned above with respect to Figures 6A and 6B, the second portion 164 of the tubular body 138 of the guiding member 136 is provided with the groove 166 of width L5 along the central longitudinal axis A of the lead 100. The width L5 of the groove 166 is substantially equal or greater than the width W2 of the second portion 180 of the sealing ring 174A. Hence, the second portion 180 of the sealing ring 174A can be received in the groove 166. In particular, the second portion 180 of the sealing ring 174A can be maintained to the groove 166 of the guiding member 136 by a form-fit connection and/or a friction-fit connection.

The second portion 164 of the tubular body 138 of the guiding member 136 is further provided with the external radially protruding shoulder 168 at the distal end 138A of the tubular body 138. The external radially protruding shoulder 168 is form-fitted into the internal radial recess 184 of width W1 of the sealing ring 174A.

As shown in **Figure 13****,** the sealing means 174 is arranged around the conductive driver shaft 120 and the tubular body 138 for sealing the distal housing portion 122 from the distal end of the lead.

Indeed, the first portion 178 of the sealing ring 174A is arranged around, in particular directly arranged around (i.e. in surface contact with), the conductive driver shaft 120. More precisely, the first portion 178 is longitudinally arranged between the external radially protruding shoulder 127 of conductive driver shaft 120 and the external radially protruding shoulder 168 at the distal end 138A of the tubular body 138.

The section of the conductive driver shaft 120 passing through the lumen 182 of the sealing ring 174A at the first portion 178 of the sealing ring 174A has an external diameter E3.

The second portion 180 of the sealing ring 174B is arranged around, in particular directly arranged around (i.e. in surface contact with) the second portion 164 of the tubular body 138 of the guiding member 136.

At the external radially protruding shoulder 168, the tubular body 138 has an external diameter E4.

The end portion housing 122, wherein at least the second portion 164 of the tubular body 138 and the conductive driver shaft 120 are received, has the internal diameter L1.

The first portion 178 of the sealing ring 174A has an external diameter E1 strictly less than the internal diameter L1 of the distal housing portion 122.

In particular, the percentage difference of diameter between the external diameter **E1** of the first portion 178 of the sealing ring 174A and the internal diameter L1 of the distal housing portion 122 is comprised between 3 % and 30%, in particular between 5% to 20%

The first portion 178 of the sealing ring 174A has an internal diameter I1 strictly less than the external diameter E3 of the conductive driver shaft 120.

In particular, the percentage difference between the internal diameter I1 of the first portion 178 of the sealing ring 174A and an external diameter E3 of the conductive driver shaft 120 is comprised between 0,05 % and 20%, in particular between 1% and 10%.

The above-mentioned range of percentage difference allows the conductive driver shaft 120 to rotate and slide with respect to the sealing ring 174A along the longitudinal axis A while preventing fluid communication at the interface between the conductive driver shaft 120 and the first portion 178 of the sealing ring 174A.

The second portion 180 of the sealing ring 174A has an external diameter E2 strictly more than the internal diameter L1 of the distal housing portion 122.

In particular, the percentage difference between the external diameter E2 of the second portion 180 of the sealing ring 174A and the internal diameter L1 of the distal housing portion 122 can be comprised between 3 % and 30%, in particular between 5% and 20%. In a preferred variant, the percentage difference between the external diameter E2 of the second portion 180 of the sealing ring 174A and the internal diameter L1 of the distal housing portion 122 is comprised between 0,5 % to 20%, in particular between 1% to 10%.

Hence, the dimensions of the sealing ring 174A of the sealing means 174 allows avoiding fluidic communication from the distal end of the lead wherein blood can enter the distal housing portion 122. It thus allows sealing the distal housing portion 122, in particular wherein the electrically conductive spring 176 is housed for providing a continuous electrical connection. The sealing means 174 thus decrease the risk of surface and/or volume corrosion inside the distal housing portion 122, in particular of the electrically conductive spring 176.

The sealing means 174 is thus designed such that its first portion 178 comprises one constrain zone at the interface between the inner wall 183 and the conductive driver shaft whereas its second portion 180 comprises two constrain zones, respectively: at the interface between the inner wall 183 and the tubular body 138, and, at the interface between the second portion 180 and the distal housing portion 122.

Hence, in contrast with the second portion 180 of the sealing means 174, there is no, or at least less, compressive forces exerted on the first portion 178 by the distal housing portion 122. This is because there is no direct surface contact between the external wall of the first portion 178 of sealing means 174 and the distal housing portion 122. Thereby, it allows reducing the friction applied by the sealing means 174 on the conductive driver shaft 120. Indeed, the first portion 178, in contrast with the second portion 180, is not squeezed (i.e. compressed or elastically deformed) and maintained between two opposite surfaces. In fact, the second portion 180 of the sealing means 174 is compressed against the internal surface of the distal housing portion 122 and the external surface of the tubular body 138.

In view of the above, the conductive driver shaft 120 can thus smoothly rotate and translate, even within the first portion 178, so as to ensure a smooth deployment of the active fixation electrode without unwanted jerky motion of the conductive driver shaft 120 caused by friction.

It is noted that the internal diameter E3 of the first portion 178 of the sealing means 174 is configured to provide sealing properties at the interface between the inner wall 183 and the tubular body 138. Hence, the sealing properties at the interface between the inner wall 183 and the tubular body 138 are ensured, despite any displacement of the conductive driver shaft 120 within the first portion 178 of the sealing means 174.

Because the second portion 180 of the sealing means 174 is radially compressed between the internal surface of the distal housing portion 122 and the external surface of the tubular body 138, the second portion 180 is maintained by form-fit and friction-fit connections between the distal housing portion 122 and the tubular body 138. It allows holding the position of the sealing means while providing sufficient sealing properties

It is noted that the Shore hardness of the sealing means 174 and the ratio of diameter differences can be advantageously selected in combination so as to provide appropriate sealing properties with minimum friction on the conductive driver shaft 120.

It is further noted that the structural features of the sealing means 174 are not intrinsically linked to the presence or not of a radial opening 158 provided at the first portion 162 of the tubular body 138 of the guiding member 136.

Although the present disclosure has been described in relation to particular examples, the invention is not limited and numerous alterations can be made without departing from the scope of this invention which is defined by the appended claims.

### Reference list

10: lead according to the first and the second examples
12: lead body
14: distal end of the lead
16: annular electrode (ring electrode)
18: coiled electrical conductors
20: sleeve
22: active fixation electrode
24: conductive driver shaft
26: internal coil conductor
34: proximal end of the sleeve 20
35: mouth piece (guiding member)
37: shoulder
48: electrically insulating sheath
50: retaining ring
54: end of the retaining ring 50
56: shoulder
58: electrically insulating adhesive
100: lead according to the third example
100A: elongated lead body
112: portion of the lead 100
114: distal end of the lead
116: coil conductor
118: lumen
120, 220: conductive driver shaft
122, 222: distal housing portion
122A: cylindrical metallic body of the distal housing portion 122
124: distal end of the conductive driver shaft
125; shoulder of the conductive driver shaft
126, 226: active fixation electrode
127: external radially protruding shoulder of the conductive driver shaft
128: ring electrode (annular electrode)
130: outer insulated sheath
132: retaining ring
134: electrically insulated sheath
136, 236: guiding member
138, 238: tubular body of the guiding member
138A: distal end
138B: proximal end
140: central lumen of the tubular body
142: annular shoulder member
144: first annular shoulder
146: second annular shoulder
148: step
150: elongated guiding portion
152: outer wall
154: annular grooves
156: seal member
158, 258: radial opening
160: chamfered edge
162: first portion
164: second portion
166: groove
168: external radially protruding shoulder
170: annular shoulder
172: junction between the first portion 162 and the second portion 164
74, 174: sealing means
174: sealing ring
176, 276: electrically conductive compression spring
176A: coil
176B: portion
178: first portion of the sealing ring
179: junction
180: second portion of the sealing ring
182: lumen of the sealing ring
183: inner wall of the sealing ring
182A: distal end of the sealing ring
182B: proximal end of the sealing ring
184: internal radial recess of the sealing ring
186A. 186B: rounded edge
188: slope
200: lead according to the fourth example
201: annular spring cover
A: central longitudinal axis of the lead 100, 200
B: central longitudinal axis of sealing ring 174A
d1: inner radius of the spring 176 at rest
d2: outer radius of the spring 176 at rest
d3: monowire thickness
d4: coil's diameter
D: direction of extension of the lead 100, 200
D2 (in the first and second examples): direction of insertion
D2 (in the third and fourth examples):
   outer diameter of the springs 176, 276 in the assembled state
E1, E2, E3, E4: external diameter
F: restoring force
I1, I2, I3: internal diameter of the sealing ring 174A
L1 (in the first and second examples):
   embodiments): distance between the cathode 22 and the anode 16
L1 (in the third and fourth examples):
   diameter of the distal housing portion 122 or of the annular spring cover 201
L2: diameter of first annular shoulder 144
L3: diameter of second annular shoulder
L4: width of the radial opening 158, 258
L5: width of the groove 166
L6: extension of the spring the direction of extension D
O: opening angle
R0: inner radius of the lumen 140
R1: outer radius of tubular body 138
T1: chamfered edge
W1, W2: width of the sealing ring 174A

## Claims

1. An implantable pacing and/or defibrillation lead, comprising a lead body (12) provided with:
a proximal end configured to be connected to an implantable medical device, and
a distal end (14), opposite to the proximal end, the distal end (14) of the lead body (12) comprising an annular electrode (16) forming an anode (16) of said lead,
the lead further comprising a sleeve (20) into which an electrode (22) forming a cathode (22) of the lead is at least partially housed, in particular said electrode (22) is a retractable fixation screw (22),
wherein the sleeve (20) is partially accommodated in the distal end (14) of the lead body (12) such that the annular electrode (16) of the lead body (12) is superimposed to the sleeve (20),
**characterized in that**
the sleeve (20) is made of a metallic material, and
the sleeve (20) is electrically insulated from the annular electrode (16) of the lead body (12) by an electrically insulating Teflon sheath (48) heat shrunk on the sleeve (20).

2. The implantable pacing and/or defibrillation lead according to one of claim 1, wherein a proximal end (34) of the sleeve (20) is accommodated within the distal end (14) of the lead body (12) and comprises a shoulder (37).

3. The implantable pacing and/or defibrillation lead according to claim 2, wherein the annular electrode (16) is attached to the sleeve (20) by clamping or crimping.

4. The implantable pacing and/or defibrillation lead according to claim 3, wherein the annular electrode (16) is clamped or crimped in at least two distinct areas of the sleeve (20).

5. The implantable pacing and/or defibrillation lead according to one of claims 1 or 2, further comprising a retaining ring (50) welded to the annular electrode (16) of the distal end (14) of the lead body (12) and arranged, along a radial direction (R) of the lead, at least partially between the annular electrode (16) and the sleeve (20), the retaining ring (50) further comprising at one end (54) thereof a shoulder (56), said shoulder (56) being arranged along a longitudinal direction (A) of the lead, between the annular electrode (16) and the sleeve (20).

6. The implantable pacing and/or defibrillation lead according to one of claims 1 to 5, wherein the annular electrode (16) has a surface of at least 15 square millimeters.

7. The implantable pacing and/or defibrillation lead according to one of claims 1 to 6, comprising an electrically insulating adhesive (58), in particular silicone adhesive or glue, between the distal end (14) of the lead body (12) and the proximal end (34) of the sleeve (20) accommodated in the distal end (14) of the lead body (12).

8. A method for assembling an implantable pacing and/or defibrillation lead according to one of claims 1 to 7, comprising the steps of:
1) Thermoforming an electrically insulating Teflon sheath (48) over a portion of the sleeve (20), and
2) Partially accommodating said portion of the sleeve (20) within the distal end (14) of the lead body (12) so that the annular electrode (16) of the lead body (12) is superimposed on the sleeve (20).

9. The method according to claim 8, further comprising a step of clamping or crimping the annular electrode (16) to the sleeve (20).

10. The method according to claim 8, wherein the step 2) further comprises:
- welding a retaining ring (50) to the annular electrode (16) of the lead body (12), and
- inserting the sleeve (20) into the lead body (12) such that the retaining ring (50) is, in a radial direction (R) of the lead, at least partially between the annular electrode (16) and the sleeve (20), and such that a shoulder (56) provided at one of the ends (54) of the retaining ring (50) is arranged, in a longitudinal direction (A) of the lead, between the annular electrode (16) and the sleeve (20).

11. The method according to claim 9 or 10, further comprising a step of introducing an electrically insulating adhesive (58) between the distal end (14) of the lead body (12) and the portion of the sleeve (20) accommodated in the distal end (14) of the lead body (12).

## Patentansprüche

1. Eine implantierbare Stimulations- und/oder Defibrillationsleitung, umfassend einen Leitungskörper (12), versehen mit:
einem proximalen Ende, das dafür ausgelegt ist, mit einem implantierbaren medizinischen Gerät verbunden zu werden, und
einem distalen Ende (14), gegenüberliegend zum proximalen Ende, wobei das distale Ende (14) des Leitungskörpers (12) eine ringförmige Elektrode (16) umfasst, die eine Anode (16) der Leitung bildet,
wobei die Leitung ferner eine Hülse (20) umfasst, in die eine Elektrode (22), die eine Kathode (22) der Leitung bildet, zumindest teilweise aufgenommen ist, insbesondere ist die Elektrode (22) eine einziehbare Fixationsschraube (22),
wobei die Hülse (20) teilweise im distalen Ende (14) des Leitungskörpers (12) aufgenommen ist, so dass die ringförmige Elektrode (16) des Leitungskörpers (12) der Hülse (20) überlagert ist,
**dadurch gekennzeichnet, dass**
die Hülse (20) aus einem metallischen Material besteht, und
die Hülse (20) elektrisch von der ringförmigen Elektrode (16) des Leitungskörpers (12) durch eine elektrisch isolierende Teflonhülle (48) isoliert ist, die auf die Hülse (20) wärmeschrumpfend aufgebracht ist.

2. Die implantierbare Stimulations- und/oder Defibrillationsleitung nach Anspruch 1, wobei ein proximales Ende (34) der Hülse (20) im distalen Ende (14) des Leitungskörpers (12) aufgenommen ist und eine Schulter (37) umfasst.

3. Die implantierbare Stimulations- und/oder Defibrillationsleitung nach Anspruch 2, wobei die ringförmige Elektrode (16) durch Klemmen oder Crimpen an der Hülse (20) befestigt ist.

4. Die implantierbare Stimulations- und/oder Defibrillationsleitung nach Anspruch 3, wobei die ringförmige Elektrode (16) in mindestens zwei unterschiedlichen Bereichen der Hülse (20) geklemmt oder gecrimpt ist.

5. Die implantierbare Stimulations- und/oder Defibrillationsleitung nach einem der Ansprüche 1 oder 2, ferner umfassend einen Haltering (50), der an die ringförmige Elektrode (16) des distalen Endes (14) des Leitungskörpers (12) angeschweißt ist und entlang einer radialen Richtung (R) der Leitung zumindest teilweise zwischen der ringförmigen Elektrode (16) und der Hülse (20) angeordnet ist, wobei der Haltering (50) an einem Ende (54) eine Schulter (56) umfasst, wobei die Schulter (56) entlang einer longitudinalen Richtung (A) der Leitung zwischen der ringförmigen Elektrode (16) und der Hülse (20) angeordnet ist.

6. Die implantierbare Stimulations- und/oder Defibrillationsleitung nach einem der Ansprüche 1 bis 5, wobei die ringförmige Elektrode (16) eine Oberfläche von mindestens 15 Quadratmillimetern aufweist.

7. Die implantierbare Stimulations- und/oder Defibrillationsleitung nach einem der Ansprüche 1 bis 6, umfassend einen elektrisch isolierenden Klebstoff (58), insbesondere Silikonkleber oder -leim, zwischen dem distalen Ende (14) des Leitungskörpers (12) und dem proximalen Ende (34) der im distalen Ende (14) des Leitungskörpers (12) aufgenommenen Hülse (20).

8. Ein Verfahren zum Zusammenbau einer implantierbaren Stimulations- und/oder Defibrillationsleitung nach einem der Ansprüche 1 bis 7, **gekennzeichnet durch** die Schritte:
1. Thermoformen einer elektrisch isolierenden Teflonhülle (48) über einen Abschnitt der Hülse (20), und
2. Teilweises Aufnehmen dieses Abschnitts der Hülse (20) im distalen Ende (14) des Leitungskörpers (12), so dass die ringförmige Elektrode (16) des Leitungskörpers (12) der Hülse (20) überlagert ist.

9. Das Verfahren nach Anspruch 8, ferner umfassend einen Schritt des Klemmens oder Crimpens der ringförmigen Elektrode (16) an der Hülse (20).

10. Das Verfahren nach Anspruch 8, wobei der Schritt 2) ferner umfasst:
- Anschweißen eines Halterings (50) an die ringförmige Elektrode (16) des Leitungskörpers (12), und
- Einführen der Hülse (20) in den Leitungskörper (12), so dass der Haltering (50) in einer radialen Richtung (R) der Leitung zumindest teilweise zwischen der ringförmigen Elektrode (16) und der Hülse (20) liegt, und so dass eine an einem der Enden (54) des Halterings (50) vorgesehene Schulter (56) in einer longitudinalen Richtung (A) der Leitung zwischen der ringförmigen Elektrode (16) und der Hülse (20) angeordnet ist.

11. Das Verfahren nach Anspruch 9 oder 10, ferner umfassend einen Schritt des Einbringens eines elektrisch isolierenden Klebstoffs (58) zwischen das distale Ende (14) des Leitungskörpers (12) und den im distalen Ende (14) des Leitungskörpers (12) aufgenommenen Abschnitt der Hülse (20).

## Revendications

1. Sonde de stimulation et/ou de défibrillation implantable, comprenant un corps de sonde (12) pourvu de :
une extrémité proximale configurée pour être connectée à un dispositif médical implantable, et
une extrémité distale (14), opposée à l'extrémité proximale, l'extrémité distale (14) du corps de sonde (12) comprenant une électrode annulaire (16) formant une anode (16) de ladite sonde,
la sonde comprenant en outre un manchon (20) dans lequel une électrode (22) formant une cathode (22) de la sonde est au moins partiellement logée, ladite électrode (22) étant en particulier une vis de fixation rétractable (22),
dans laquelle le manchon (20) est partiellement logé dans l'extrémité distale (14) du corps de sonde (12) de sorte que l'électrode annulaire (16) du corps de sonde (12) est superposée au manchon (20),
**caractérisée en ce que :**
le manchon (20) est constitué d'un matériau métallique, et
le manchon (20) est électriquement isolé de l'électrode annulaire (16) du corps de sonde (12) par une gaine électriquement isolante en Téflon (48) thermorétractée sur le manchon (20).

2. Sonde de stimulation et/ou de défibrillation implantable selon la revendication 1, dans laquelle une extrémité proximale (34) du manchon (20) est logée dans l'extrémité distale (14) du corps de sonde (12) et comprend un épaulement (37).

3. Sonde de stimulation et/ou de défibrillation implantable selon la revendication 2, dans laquelle l'électrode annulaire (16) est fixée au manchon (20) par sertissage ou pincement.

4. Sonde de stimulation et/ou de défibrillation implantable selon la revendication 3, dans laquelle l'électrode annulaire (16) est sertie ou pincée en au moins deux zones distinctes du manchon (20).

5. Sonde de stimulation et/ou de défibrillation implantable selon l'une quelconque des revendications 1 ou 2, comprenant en outre une bague de retenue (50) soudée à l'électrode annulaire (16) de l'extrémité distale (14) du corps de sonde (12) et disposée, selon une direction radiale (R) de la sonde, au moins partiellement entre l'électrode annulaire (16) et le manchon (20), la bague de retenue (50) comprenant en outre, à l'une de ses extrémités (54), un épaulement (56), ledit épaulement (56) étant disposé, selon une direction longitudinale (A) de la sonde, entre l'électrode annulaire (16) et le manchon (20).

6. Sonde de stimulation et/ou de défibrillation implantable selon l'une quelconque des revendications 1 à 5, dans laquelle l'électrode annulaire (16) présente une surface d'au moins 15 millimètres carrés.

7. Sonde de stimulation et/ou de défibrillation implantable selon l'une quelconque des revendications 1 à 6, comprenant un adhésif électriquement isolant (58), en particulier un adhésif ou une colle à base de silicone, entre l'extrémité distale (14) du corps de sonde (12) et l'extrémité proximale (34) du manchon (20) logée dans l'extrémité distale (14) du corps de sonde (12).

8. Procédé d'assemblage d'une sonde de stimulation et/ou de défibrillation implantable selon l'une quelconque des revendications 1 à 7, **caractérisé par** les étapes consistant à :
1) thermoformer une gaine isolante en Téflon (48) sur une portion du manchon (20) ; et
2) loger partiellement ladite portion du manchon (20) dans l'extrémité distale (14) du corps de sonde (12) de sorte que l'électrode annulaire (16) du corps de sonde (12) soit superposée au manchon (20).

9. Procédé selon la revendication 8, comprenant en outre une étape de sertissage ou de pincement de l'électrode annulaire (16) sur le manchon (20).

10. Procédé selon la revendication 8, dans lequel l'étape 2) consiste en outre à :
- souder une bague de retenue (50) à l'électrode annulaire (16) du corps de sonde (12) ; et
- insérer le manchon (20) dans le corps de sonde (12) de telle sorte que la bague de retenue (50) soit, selon une direction radiale (R) de la sonde, au moins partiellement entre l'électrode annulaire (16) et le manchon (20), et de telle sorte qu'un épaulement (56) prévu à l'une des extrémités (54) de la bague de retenue (50) soit disposé, selon une direction longitudinale (A) de la sonde, entre l'électrode annulaire (16) et le manchon (20).

11. Procédé selon la revendication 9 ou 10, comprenant en outre une étape d'introduction d'un adhésif électriquement isolant (58) entre l'extrémité distale (14) du corps de sonde (12) et la portion du manchon (20) logée dans l'extrémité distale (14) du corps de sonde (12).
